(19) Europäisches Patentamt
European Patent Office
Office européen des brevets

(11) **EP 4 365 284 A1**

(12) **EUROPEAN PATENT APPLICATION**
published in accordance with Art. 153(4) EPC

(43) Date of publication:
**08.05.2024 Bulletin 2024/19**

(21) Application number: 22833318.3

(22) Date of filing: **30.06.2022**

(51) International Patent Classification (IPC):
*C12N 7/02* (2006.01)    *B01D 61/00* (2006.01)
*B01D 69/08* (2006.01)    *C12N 1/00* (2006.01)
*C12N 1/02* (2006.01)

(52) Cooperative Patent Classification (CPC):
**B01D 61/00; B01D 69/08; C12N 1/00; C12N 1/02; C12N 7/02**

(86) International application number:
**PCT/JP2022/026434**

(87) International publication number:
**WO 2023/277173 (05.01.2023 Gazette 2023/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 30.06.2021  JP 2021108995
30.06.2021  JP 2021109004

(71) Applicant: **Asahi Kasei Medical Co., Ltd.**
**Tokyo 100-0006 (JP)**

(72) Inventors:
• **MIYAOKA, Rimi**
**Tokyo 100-0006 (JP)**
• **TANIGUCHI, Hiroki**
**Tokyo 100-0006 (JP)**
• **OKADA, Takashi**
**Tokyo 108-0071 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **VIRUS RECOVERY METHOD**

(57)    The purpose of the invention is to provide a virus recovery method including reducing viability of cells in a culture solution and filtering, through a hollow fiber membrane, the culture solution containing a virus produced by the cells to recover the virus. The hollow fiber membrane has a gradient structure in which an average pore size becomes smaller from an upstream side to a downstream side in a membrane thickness direction.

Fig. 1

## Description

### Technical Field

The present invention relates to a filtration method.

### Background Art

Cell culture technology is indispensable for the production of various biopharmaceuticals such as viruses including virus vectors, antibodies, growth hormones, and insulin and it largely contributes to the recent progress in medicine. Among the biopharmaceuticals, virotherapeutic agents or virus vaccines have particularly attracted attentions. Highly efficient and stable production of virotherapeutic agents or virus vaccines by culturing virus-producing cells is one of industrially important themes (refer to, for example, Patent Documents 1 to 4).

For the production of a virus to be used for biopharmaceuticals, it is necessary to culture virus-producing cells, remove the virus-producing cells from a cell culture solution, and purify the virus. Examples of the conventional method for removing cells from a cell culture solution include a gel filtration method, a centrifugal separation method, an adsorptive separation method, a precipitation method, and a membrane filtration method. However, the gel filtration method is accompanied with the problems that a material to be purified is inevitably diluted with a solvent used for gel filtration and the method is not suited for large-scale processing so that industrial use of this method is difficult. The centrifugal separation method can only be applied to a solution having a small viscosity and large-scale equipment is difficult to install. The precipitation method has the problem that single use of it cannot remove the cells completely.

Citation List

Patent Documents

Patent Document 1: Japanese Unexamined Patent Publication No. 2018-076291
Patent Document 2: Japanese Patent No. 6530171
Patent Document 3: International Publication No. 2010/035793
Patent Document 4: International Publication No. 2020/023612

### Summary of Invention

Technical Problem

The membrane filtration method using a microfiltration membrane or ultrafiltration membrane easily removes cells and at the same time can continuously process a large amount of a solution so that it is suited for industrial use. The conventional membrane filtration method however has the problem that a concentrated layer of cells or cell debris is formed on the membrane surface to clog the membrane surface therewith and causes an increase in filtration pressure and a decrease in filtration rate with time. In particular, when cells contained in a cell culture solution have low viability and the cell culture solution contains many dead cells, this method has the problem that a lot of cell debris exists in the cell culture solution and the cell debris accumulated on the membrane surface or inside the membrane prevents permeation of the material to be purified and reduces the recovery percent of the material to be purified.

According to the finding of the present inventors, the viability of cells decreases with the passage of culture time. An amount of a cell-producing material however increases with the passage of culture time. In addition, an amount of the cell producing material may increase at the timing when the viability decreases. In addition, according to the finding of the present inventors, when the cell producing material is accumulated inside the cell, for example, culturing for long hours may reduce the cell viability and release the cell producing material out of the cells. According to the finding of the present inventors, however, there is a problem that a filtration processing amount is lower, in other words, a filtration efficiency is lower for a cell culture solution containing cells having low viability than for a cell culture solution containing cells having high viability.

In addition, the conventional filtration membrane has the problem that the continuous use of it reduces the permeability of a material to be purified. The reduction in the permeability of a material to be purified through a filtration membrane may reduce the recovery percent of the material to be purified and increase a ratio of impurities such as protein aggregate. The reduction in the recovery percent of the material to be purified may lead to a steep rise in a production cost of pharmaceuticals using a cell producing material as a raw material and a steep rise in medical expenses.

One of the objects of the present invention is therefore to provide a virus recovery method with a high recovery percent. For example, one of the objects of the present invention is to provide a method of filtering, with good virus permeability, a virus-containing culture solution.

Solution to Problem

The virus recovery method according to an aspect of the present invention includes reducing the viability of cells in a culture solution and filtering the culture solution containing a virus produced by the cells by using a hollow fiber membrane to recover the virus. The hollow fiber membrane has a gradient structure in which an average pore size becomes smaller from an upstream side to a downstream side in the membrane thickness direction. The filtration may be tangential flow filtration.

In the aforesaid virus recovery method, cells may be removed by filtration.

In the aforesaid virus recovery method, cell debris may be removed by filtration.

In the aforesaid virus recovery method, an upstream side pore size of the hollow fiber membrane may be 20 um or more and 100 $\mu$m or less.

In the aforesaid virus recovery method, a blocking pore size of the pore which the hollow fiber membrane has may be 0.05 um or more to 20 $\mu$m or less.

In the aforesaid virus recovery method, the hollow fiber membrane may be made of a synthetic polymer.

In the aforesaid virus recovery method, the synthetic polymer may be polysulfone.

In the aforesaid virus recovery method, the hollow fiber membrane may have a coarse layer or a dense layer.

In the aforesaid virus recovery method, in reducing viability of cells, the viability of cells may be reduced by chemical processing or physical processing.

In the aforesaid virus recovery method, the viability of cells may be reduced by bringing the cells into contact with a chemical material.

In the aforesaid virus recovery method, the chemical material may be a surfactant, an acidic material, or a basic material.

In the aforesaid virus recovery method, the cells may be lysed by the surfactant.

In the aforesaid virus recovery method, the surfactant may be a nonionic surfactant or an amphoteric surfactant.

In the aforesaid virus recovery method, the concentration of the nonionic surfactant in the culture solution may be 0.005% or more, 0.01% or more, 0.02% or more, 0.03% or more, 0.04% or more, 0.05% or more, 0.06% or more, 0.07% or more, 0.08% or more, 0.09% or more, or 0.1% or more.

In the aforesaid virus recovery method, the concentration of the nonionic surfactant in the culture solution may be 1% or less, 0.9% or less, 0.8% or less, 0.7% or less, 0.6% or less, 0.5% or less, 0.4% or less, 0.3% or less, 0.2% or less, or 0.1% or less.

In the aforesaid virus recovery method, the concentration of the amphoteric surfactant in the culture solution may be 0.01% or more, 0.02% or more, 0.04% or more, 0.06% or more, 0.08% or more, 0.1% or more, 0.2% or more, 0.3% or more, 0.4% or more, or 0.5% or more.

In the aforesaid virus recovery method, the concentration of the amphoteric surfactant in the culture solution may be 2.0% or less, 1.8% or less, 1.6% or less, 1.4% or less, 1.2% or less, 1.0% or less, 0.8% or less, 0.6% or less, or 0.5% or less.

In the aforesaid virus recovery method, the viability of cells may be reduced by transfecting a chemical material into the cells.

In the aforesaid virus recovery method, the physical processing may include cell disruption.

In the aforesaid virus recovery method, cells after reduction of the viability of cells may have viability of 60% or less.

In the aforesaid virus recovery method, cells after reduction of viability of cells may have viability of 30% or less.

In the aforesaid virus recovery method, the culture solution before reduction of the viability of cells may have a cell density of $1.0 \times 10^5$ cells/mL or more, $2.0 \times 10^5$ cells/mL or more, $4.0 \times 10^5$ cells/mL or more, $6.0 \times 10^5$ cells/mL or more, $8.0 \times 10^5$ cells/mL or more, or $1.0 \times 10^6$ cells/mL or more.

In the aforesaid virus recovery method, the culture solution before reduction of the viability of cells may have a cell density of $1.0 \times 10^8$ cells/mL or less, $8.0 \times 10^7$ cells/mL or less, $6.0 \times 10^7$ cells/mL or less, $4.0 \times 10^7$ cells/mL or less, $2.0 \times 10^7$ cells/mL or less, $1.0 \times 10^7$ cells/mL or less, $8.0 \times 10^6$ cells/mL or less, $6.0 \times 10^6$ cells/mL or less, $4.0 \times 10^6$ cells/mL or less, or $2.0 \times 10^6$ cells/mL or less.

In the aforesaid virus recovery method, the cells may be genetically modified cells.

In the aforesaid virus recovery method, the virus may be a non-enveloped virus.

In the aforesaid virus recovery method, the virus may be a parvovirus.

In the aforesaid virus recovery method, the virus is an adeno-associated virus.

In the aforesaid virus recovery method, the virus may be an enveloped virus.

In the aforesaid virus recovery method, the virus may be a retrovirus.

In the aforesaid virus recovery method, the virus may be a lentivirus.

The aforesaid virus recovery method may not substantially include, before the filtering, removing cells and/or debris of the cells with a precipitating medium.

Advantageous Effects of Invention

According to the present invention, a virus recovery method with a high recovery percent can be provided.

**Brief Description of Drawings**

Fig. 1 is a graph showing AAV permeability according to Example.

Fig. 2 is a graph showing HCP permeability according to Example.

**Description of Embodiments**

The embodiment of the present invention (which may hereinafter be shortened to "the embodiment") will hereinafter be described in detail. The following embodiments exemplify a method or the like for embodying the technical concept of the present invention but the invention is not limited by them.

A virus recovery method according to the embodiment includes reducing the viability of cells in a culture solution and filtering the culture solution containing a virus produced by the cells through a hollow fiber membrane to recover the virus. The hollow fiber membrane has a gradient structure in which an average pore size thereof becomes smaller from an upstream side to a downstream side in a membrane thickness direction.

The step of reducing viability of cells is not particularly limited insofar as it is a step of reducing viability of cells in a cell culture solution by a positive action and examples include a step of reducing viability of cells by chemical processing or physical processing. For example, simple culturing of cells for long hours includes neither chemical processing nor physical processing and therefore does not correspond to the positive action for reducing viability of cells. On the other hand, for example, transfection is a positive cell viability reduction step that causes cells to produce a material which may have cytotoxicity and thereby reduces the cell viability. It is an example of one aspect of the step of reducing viability of cells by chemical processing. Cell disruption processing is chemical processing or physical processing capable of damaging a cell membrane. Although either physical processing or chemical processing may be used as a viability reduction method, chemical processing which facilitates scale-up is preferred.

The chemical processing according to the embodiment includes addition of a chemical material. No limitation is imposed insofar as the chemical material reduces viability. As the chemical material, at least any of factors, such as nucleic acid for producing a useful material, vectors, and transfection reagents, to be introduced into cells at the time of transfection can be given as examples. Examples of another aspect of the chemical material include surfactants, acidic materials, and basic materials. The acidic materials may be in an acidic solution form obtained by dissolving it in a solvent (water or alcohol can be given as an example) . The basic material may be in a basic solution form obtained by dissolving it in a solvent (water or alcohol can be given as an example). Examples of the acidic material include hydrochloric acid, sulfuric acid, nitric acid, and acetic acid. Examples of the basic material include sodium hydroxide, potassium hydroxide, calcium hydroxide, magnesium hydroxide, and ammonia.

The cells may be either those derived from animals including humans or those derived from microorganisms. Examples of the animals include mammals, reptiles, birds, amphibians, fishes, and insects. The cells are, for example, those producing a useful material, but not limited thereto. The cells may be genetically modified cells. Examples of the cells include, but not limited to, HEK293 cells, HEK911 cells, PER.C6 cells, Sf9 cells, and CHO (Chinese Hamster Ovary) cells.

The virus includes virus-like particles (VLP). The virus includes a virus vector. The virus includes an oncolytic virus. The virus includes a virus vaccine. The virus vaccine includes a bioengineered vaccine. The virus is classified into a virus released from cells into a cell culture solution and a virus accumulated inside cells.

The virus may be either a non-enveloped virus or an enveloped virus. Examples of the non-enveloped virus include, but not limited to, adenovirus, adeno-associated virus (AAV), and parvovirus. The AAV has a weaker electric charge than the adenovirus and is likely to adsorb to a hollow fiber membrane. Examples of the enveloped virus include, but not limited to, retrovirus, lentivirus, Sendai virus, rabies virus, sindbis virus, and herpes simplex virus.

The cells may be, for example, transfected. By the transfection, a factor, such as nucleic acid, that produces a virus is introduced into cells. During transfection, cell viability decreases. The factor such as nucleic acid is a chemical material. The introduction of the factor by transfection includes, for example, introduction of the factor by electroporation, introduction of the factor by lipofection, and introduction of the factor by a virus factor.

The cells may be cultured either by an adherent culture method or a suspension culture (floating culture) method. In the adherent culture method, cells adhere to the inner surface of a culture tank. In the suspension culture method, cells are suspended in a cell culture solution.

In the method of culturing cells by suspension culture, for example, a stirring mechanism is provided in a culture tank such as spinner flask to suspend cells. As the stirring mechanism, used is a magnetic stirrer or a mechanically driven shaft-like impeller. A method of, during suspension culture, culturing cells while supplying a fresh medium in the culture tank and discharging an old cell culture solution containing an impurity such as growth inhibiting material out of the culture tank is called "continuous culture".

The culture method may be any of a batch culture method, a fed-batch culture method, or a continuous culture method. The batch culture method is a method of culturing without supplying a new cell culture solution in a culture tank. The fed-batch culture method is a method of culturing by supplying a new cell culture solution in a culture tank. The continuous culture method is a method of culturing by supplying a new cell culture solution in a culture tank and discharging an old cell culture solution from the culture tank, while keeping the amount of the cell culture solution in the culture tank constant. The step of reducing viability may be performed after culturing or during culturing. The filtration operation may be performed after culturing or continuously during culturing.

When the virus is recovered from the cells, the cells may be disrupted. By the disruption of the cells, the virus in the cells diffuse in the cell culture solution. At the time of disruption of the cells, the viability of the cells decreases. A chemical material such as surfactant, acidic material, or basic material may be added to the cells to carry out cytolytic chemical processing. It is to be noted that cytolysis is not necessary for an enveloped virus, because the virus is extracellularly produced. In general, the non-enveloped virus is intracellularly produced, but the adeno-associated viruses (AAV) such as AAV8 and AAV9 are extracellularly produced, though depending on the serotype (serum type). In this case, cytolysis is not always required.

The surfactant may be either a nonionic surfactant or an amphoteric surfactant. A nonionic surfactant may be mixed with an amphoteric surfactant at an appropriate concentration. The surfactant may be present in an appropriate solvent. In general, the nonionic surfactant and the amphoteric surfactant are less likely to modify a protein than an ionic surfactant.

Examples of the nonionic surfactant include, but not limited to, Triton X-100, Triton X-114, NP-40, Brij-35, Brij-58, Tween-20, Tween-80, octyl glucoside, and octylthio glucoside.

Examples of the amphoteric surfactant include, but not limited to, CHAPS

(3-[(3-cholamidopropyl)dimethylammonio]propanesulfonate) and CHAPSO

(3[(3-cholamidopropyl)dimethylammonio]-2-hydroxypropanesulfoni c acid).

The cell density in the cell culture solution before disruption of the cells is, for example, $1.0 \times 10^5$ cells/mL or more, $2.0 \times 10^5$ cells/mL or more, $4.0 \times 10^5$ cells/mL or more, $6.0 \times 10^5$ cells/mL or more, $8.0 \times 10^5$ cells/mL or more, or $1.0 \times 10^6$ cells/mL or more and at the same time, $1.0 \times 10^8$ cells/mL or less, $8.0 \times 10^7$ cells/mL or less, $6.0 \times 10^7$ cells/mL or less, $4.0 \times 10^7$ cells/mL or less, $2.0 \times 10^7$ cells/mL or less, $1.0 \times 10^7$ cells/mL or less, $8.0 \times 10^6$ cells/mL or less, $6.0 \times 10^6$ cells/mL or less, $4.0 \times 10^6$ cells/mL or less, or $2.0 \times 10^6$ cells/mL or less. The cell density in the cell culture solution can be measured with an automated cell counter CYTORECON (product of GE Healthcare).

When the cell density in the cell culture solution is as described above, the concentration of the nonionic surfactant in the cell culture solution is, for example, preferably 0.005% or more and from the standpoint of releasing the material from the cells, it is more preferably 0.01% or more, 0.02% or more, 0.03% or more, 0.04% or more, 0.05% or more, 0.06% or more, 0.07% or more, 0.08% or more, 0.09% or more, or 0.1% or more. Further, it is preferably 1% or less. From the standpoint of removing the used surfactant in a downstream purification step, it is more preferably 0.9% or less, 0.8% or less, 0.7% or less, 0.6% or less, 0.5% or less, 0.4% or less, 0.3% or less, 0.2% or less, or 0.1% or less.

When the cell density in the cell culture solution is as described above, the concentration of the amphoteric surfactant in the cell culture solution is, for example, preferably 0.01% or more and from the standpoint of releasing the material from the cells, it is more preferably 0.02% or more, 0.04% or more, 0.06% or more, 0.08% or more, 0.1% or more, 0.2% or more, 0.3% or more, 0.4% or more, or 0.5% or more. Further, it is preferably 2.0% or less. From the standpoint of removing the used surfactant in the downstream purification step, it is more preferably 1.8% or less, 1.6% or less, 1.4% or less, 1.2% or less, 1.0% or less, 0.8% or less, 0.6% or less, or 0.5% or less.

The cell disruption method is not limited to chemical processing. For example, cells may be disrupted using an enzyme. Cells may be disrupted by mechanical or physical processing that applies a pressure to the cells. The cells may be disrupted using a machine such as high-pressure homogenizer or mill. The pressure may be a pressure by ultrasonic waves, cavitation, or osmotic pressure. The cells may be disrupted by heat processing that applies heat thereto. Or, the cells may be disrupted by applying a thermal shock to the cells by a freeze-melt method. The cells thus disrupted may be retained by batch culture that does not supply a new cell culture solution in the culture tank.

In one mode, a cell-produced material (virus) may be produced in the following order: cell culturing, transfection, cell culturing, processing of the cells with a surfactant, and batch filtration of the cell culture solution. In another mode, a cell-produced material may be produced in the following order: cell culturing, transfection, cell culturing, and batch filtration of the cell culture solution. In a further mode, a cell-produced material may be produced in the following order: cell culturing, transfection, and continuous culturing.

The term "viability of cells" means a ratio of the number of living cells to the total number of cells in the solution. In one mode, the viability of cells before one step of reducing the viability of cells is, for example, 60% or more, 65% or more, 70% or more, 75% or more, 80% or more, 85% or more, 90% or more, 95% or more, or 99% or more; and the viability of cells immediately before filtration but after one step of reducing the viability of cells is, for example, 50% or less, 45% or less, 40% or less, 35% or less, 30% or less, 25% or less, 20% or less, 15% or less, 10% or less, or 5% or less. The term "one step of reducing the viability of cells" as used herein means, for example, the aforesaid transfection, but is not limited thereto.

In one mode, the viability of cells before one step of reducing the viability of cells is, for example, 30% or more, 35% or more, 40% or more, 45% or more, 50% or more, 55% or more, 60% or more, 65% or more, or 70% or more; and the viability of cells immediately before filtration but after one step of reducing the viability of cells is, for example, 25% or less, 20% or less, 15% or less, 10% or less, or 5% or less. The term "one step of reducing the viability of cells" as used herein means, for example, the aforesaid cell disruption processing and at the same time, a step after transfection, but the step is not limited thereto.

In one mode, the viability of cells before one step of reducing the viability of cells is, for example, 80% or more, 85% or more, 90% or more, 95% or more, or 99% or more; and the viability of cells immediately before filtration but after one step of reducing the viability of cells is, for example, 75% or less, 70% or less, 65% or less, 60% or less, 55% or less, 50% or less, 45% or less, 40% or less, 35% or less, 30% or less, 25% or less, 20% or less, 15% or less, 10% or less, or 5% or less. The term "one step of reducing the viability of cells" as used herein means the aforesaid transfection used in combination with continuous culture, but it is not limited thereto.

When there are two or more steps of reducing the viability of cells, the aforesaid term "viability of cells" means viability of cells before and after one step of reducing the viability of cells which is proximate to a step of filtering the cell culture solution through a hollow fiber membrane.

The viability of cells can be measured with an automated cell counter "Vi-CELL XR", product of Beckman Coulter).

The culture tank for having therein a cell culture solution containing cells to be filtered may have an outlet for feeding an upstream side surface of the hollow fiber membrane with the cell culture solution and an inlet for returning, to the culture tank, the cell culture solution which has not permeated through the inside of the hollow fiber membrane but passed the upstream side surface of the hollow fiber membrane. These outlet and inlet may be the same or different from each other.

The surface of the hollow fiber membrane to which the cell culture solution to be filtered is supplied is called "upstream side". The surface of the hollow fiber membrane from which a permeate which has passed the hollow fiber membrane comes is called "downstream side".

In an embodiment in which a cell culture solution to be filtered is supplied to the inner circumferential surface, the inner circumferential surface of the hollow fiber membrane is called "an upstream side", while the outer circumferential surface of the hollow fiber membrane is called "a downstream side". In an embodiment in which a cell culture solution to be filtered is supplied to the outer circumferential surface, the outer circumferential surface of the hollow fiber membrane is called "an upstream side", while the inner circumferential surface of the hollow fiber membrane is called "a downstream side".

A plurality of hollow fiber membranes is incorporated, for example, in a filtration module. The filtration module may have a cell culture solution inlet which is an inlet from which the cell culture solution fed from the culture tank enters and is communicated with the upstream side surface of the hollow fiber membrane; and a cell culture solution outlet for returning, to the culture tank, the cell culture solution which has not permeated through the inside of the hollow yarn membrane but has passed the upstream side surface of the hollow fiber membrane. The cell culture solution inlet and the cell culture solution outlet may be the same or different from each other. The filtration module may have a permeate outlet for allowing a virus-containing permeate, which has permeated through the inside of the hollow fiber membrane and has flown out from the downstream side surface of the hollow fiber membrane, to flow out.

The culture tank and the filtration module are connected to each other, for example, by a liquid feed mechanism equipped with a flow channel and a pump. The liquid feed mechanism may be equipped with a pressure gauge and a gravimeter. Examples of the pump include, but not limited to, a diaphragm pump, a tube pump, and a rotary pump.

The cell culture solution having cultured cells therein is filtered through a hollow fiber membrane to remove the cells from the cell culture solution, permeate the virus in the cell culture solution through the hollow fiber membrane, and purify it.

Filtration may be tangential flow filtration. The term "tangential flow filtration (TFF) method" is a filtration method of allowing the cell culture solution to flow on the upstream side surface of the hollow fiber membrane in a direction parallel to the upstream side surface of the hollow fiber membrane. The tangential flow filtration (TFF) method includes an alternating tangential flow filtration (ATF) method. In the present embodiment, when the simple term "tangential flow filtration (TFF) method" is used, it sometimes means a filtration method in which the cell culture solution is allowed to flow in one direction at the hollow portion of the hollow fiber membrane. The term "alternating tangential flow filtration (ATF) method" means a filtration method in which the cell culture solution is allowed to flow back and forth at the hollow portion of the hollow fiber membrane.

The porous structure of the hollow fiber membrane has a gradient structure as a whole. In the hollow fiber membrane having a gradient structure, the average pore size of the upstream side surface is larger than the average pore size of the downstream side surface and the pore size becomes smaller from the upstream side surface to a minimum pore size layer. There may be a portion of the membrane where the pore size does not change between the upstream side surface and the downstream side surface. In the hollow fiber membrane having a gradient structure, therefore, the pore size distribution is asymmetric in the membrane thickness direction. A layer having a relatively large pore size in the vicinity of the upstream side surface of the hollow fiber membrane is called "a coarse layer". A layer having a relatively small pore size in the vicinity of the downstream side surface of the hollow fiber membrane is called "a dense layer". The minimum pore size layer having a minimum pore size is included in the dense layer.

The average pore size of the upstream side surface in the hollow fiber membrane having a gradient structure is, for example, 1 um or more, 10 um or more, 20 um or more, or 30 um or more and 100 $\mu$m or less, 90 $\mu$m or less, or 80 um or more. When the upstream side surface has a pore size of 1 um or more, the effect of the depth filtration on the retention of the removed material inside the membrane tends to be easier to obtain and at the same time, the pore of the membrane tends to be less likely to be clogged with the removed material deposited on the membrane surface. The hollow fiber membrane tends to keep its strength when the pore size of the upstream side surface is 100 $\mu$m or less.

The average pore size of the downstream side surface in the hollow fiber membrane having a gradient structure is, for example, 0.1 um or more and 20 $\mu$m or less, 0.2 um or more and 15 $\mu$m or less, or 0.3 um or more and 10 $\mu$m or less.

The average pore size of the surface of the hollow fiber membrane is determined by freeze drying the hollow fiber membrane and observing, with an electron microscope (VE-9800, product of Keyence), 10 or more pores in one visual field. The 10 pores thus observed are each subjected to circular contour approximation and an average of the diameters determined from the area of the 10 pores approximated to a circle is taken as an average pore size.

The blocking pore size of the hollow fiber membrane having a gradient structure is, for example, 0.05 um or more, 0.1 um or more, 0.2 $\mu$m or more, or 0.3 $\mu$m or more; and the blocking pore size of the hollow fiber membrane having a gradient structure is, for example, 20 $\mu$m or less, 10 $\mu$m or less, 5 $\mu$m or less, 3 $\mu$m or less, 1 um or less, 0.8 um or less, or 0.5 um or less. The blocking pore size of 0.05 um or more tends to suppress a permeation resistance, suppress a pressure necessary for filtration, and suppress the clogging of the membrane surface and a reduction in filtration efficiency due to destruction and deformation of microorganism particles. When the blocking pore size is 20 $\mu$m or less, on the other hand, a sufficient fractionation property tends to be obtained.

The term "blocking pore size" is exemplified as a pore size of particles at which, when a particle dispersion having particles having a predetermined pore size dispersed therein is filtered through a porous hollow fiber membrane, a permeation blocking rate of the particles is 90%. It may also be called "a minimum pore size" . In determining the blocking pore size, polystyrene latex particles (SIZE STANDARD PARTICLES, product of JSR) are dispersed in a 0.5 mass% aqueous sodium dodecyl sulfate (product of Wako Pure Chemical) solution to give a particle concentration of 0.01 mass% and thus, a latex particle dispersion is prepared. The latex particle dispersion is filtered using the porous hollow fiber membrane and a concentration change of the latex particles before and after filtration is measured. This measurement is performed while changing the latex particle size from 0.1 um in about 0.1 um increments to create a blocking curve of the latex particles. From this blocking curve, a particle size capable of blocking 90% of permeation is read and the size is defined as a blocking pore size.

The hollow fiber membrane having a gradient structure may include, in the vicinity of the downstream side surface, the minimum pore size layer in which the pore size is minimum. The minimum pore size of the hollow fiber membrane is almost equal to the blocking pore size.

The inner diameter of the hollow portion of the hollow fiber membrane is, for example, 1000 um or more and 2000 $\mu$m or less, 1000 um or more and 1500 $\mu$m or less, or 1100 um or more and 1400 $\mu$m or less. When the hollow portion has an inner diameter of 1000 um or more, the entrance of the hollow portion tends not to be blocked with cells. When the hollow portion has an inner diameter of 2000 um or less, the number of the hollow fiber membranes constituting the filtration module increases, an effective cross-sectional area per filtration module becomes large, and filtration performance tends to be excellent.

The thickness of the hollow fiber membrane is, for example, 300 um or more and 1000 $\mu$m or less, 350 um or more and 800 $\mu$m or less, or 350 um or more and 500 um or less. The hollow fiber membrane having a thickness of 300 um or more can retain a removed material inside the membrane and therefore tends to produce a depth filtration effect.

Further, it tends to be likely to keep an appropriate filtration rate. The hollow fiber membrane having a thickness of 1000 um or less tends to be excellent in filtration performance because the number of the hollow fiber membranes constituting the filtration module increases and an effective cross-sectional area per filtration module becomes larger.

The inner diameter and outer diameter ($\mu$m) of the hollow fiber membrane can be measured by slicing the hollow fiber membrane into a thin round tube and observing the resulting section with an optical microscope (VH6100, product of Keyence) . The thickness $T_h$ ($\mu$m) of the hollow fiber membrane can be calculated by the following equation based on the inner diameter $D_I$ and the outer diameter $D_O$:

$$T_h = (D_O - D_I) / 2$$

Virus permeability can be measured by quantitatively determining, by quantitative PCR, a virus concentration in the permeate after the filtration step and a virus concentration in the cell culture solution or cell lysate before the filtration step and comparing them. For example, the virus permeability can be calculated using the following equation:

Permeability X = (virus concentration in permeate) / (virus concentration in cell culture solution or cell lysate before filtration) $\times$ 100

The virus concentration is determined by measuring a virus amount. The virus amount can be measured by real-time PCR. When the virus is AAV, AAV pro Titration kit (Takara Bio) is usable as a reagent for measuring the amount of AAV. A culture solution or culture lysate before filtration is centrifuged at 300$\times$g for 2 minutes and then a supernatant is sampled.

The HPLC measurement of an antibody concentration is performed by the following method.

(1) Detector: ultraviolet absorptiometer (measurement wavelength: 280 nm)
(2) Column: POROS G 20 um Column, 4.6$\times$50 mm, 0.8 mL (Thermo Fisher)
(3) Column temperature: room temperature
(4) Mobile phase

Mobile phase A: 7.098 g of disodium hydrogen phosphate (anhydrous) and 8.766 g of sodium chloride are dissolved in 800 mL of water. After addition of 1 mol/L hydrochloric acid to adjust the pH to 7.0, water is added to make 1000 mL.

Mobile phase B: 12 mL of 1 mol/L hydrochloric acid and 8.766 g of sodium chloride are dissolved in water to make 1000 mL.

(5) Feeding of mobile phase

The mobile phase A and the mobile phase B are fed at a flow rate of 2 ml/min while changing their ratio as described in the following table.

[Table 1]

| Time (min) after sample injection | Mobile phase A (vol%) | Mobile phase B (vol%) |
| --- | --- | --- |
| 0 to 4 | 100 | 0 |
| 4 to 11 | 0 | 100 |
| 11 to 16 | 100 | 0 |

After the cell culture solution and cell lysate are centrifuged at 300$\times$g for 2 minutes, the supernatants are sampled, respectively. Nine serially diluted solutions of a commercially-available human immunoglobulin G (blood donation Venoglobulin IH5% for intravenous injection 2.5 g/50 ml, product of Japan Blood Products Organization), the permeate, the supernatant of the cell culture solution, and the supernatant of the cell lysate are fed in the aforesaid order by the aforesaid method. A standard curve is created using nine peak areas of human immunoglobulin G and then, from the standard curve and peak area of the sample, the antibody concentration in each solution is calculated.

The hollow fiber membrane is for example made of a synthetic polymer membrane. The synthetic polymer is, for example, hydrophobic. Examples of the synthetic polymer include, but not limited to, polysulfone. In general, cells or impurities such as debris (cell debris) contained in the cell culture solution are hydrophobic. Due to the hydrophobic interaction, the impurities are captured by the hollow fiber membrane. This makes it possible to purify the cell culture solution containing a useful material. The hollow fiber membrane according to the embodiment can be produced referring to, for example, the method described in International Publication No. 2010/035793.

The virus recovery method of the present embodiment may include removal of cells and cell debris before filtration, but the virus recovery method of the present embodiment may not substantially include removal of cells and cell debris before filtration because it achieves highly efficient virus recovery. Examples of the method of removing cells and cell debris include removal of cells and cell debris by means of a precipitating medium and removal of cells and cell debris by centrifugal separation. At the time of removing cells and cell debris, an impurity protein and DNA may be removed together.

The turbidity in the solution can be measured with a portable turbidimeter (portable turbidimeter 210Q, product of Fuji Tecom).

Although not bound by a theory, a reduction in a filtration processing amount by the hollow fiber membrane can be suppressed by making clogging of the hollow fiber membrane less likely to occur. For example, there is a possibility of the turbidity of the cell culture solution or the size of particles present in the cell culture solution being involved in the clogging of the hollow fiber membrane. In a certain embodiment, a reduction in the filtration processing amount by the hollow fiber membrane can be suppressed by decreasing the turbidity of the cell culture solution before filtration of the cell culture solution. In another embodiment, a reduction in the filtration processing amount by the hollow fiber membrane can be suppressed by decreasing the size of the particles parent in the cell culture solution before filtration of the cell culture solution.

Examples

The present invention will hereinafter be described in detail by Referential Examples, Referential Comparative Examples, Experiment Examples, Examples, and Comparative Examples, but the present invention is not limited by the following Examples, Comparative Examples, and Experiment Examples. Test methods in Referential Examples, Referential Comparative Examples, Experiment Examples, Examples, and Comparative Examples are as described below.

(Referential Example 1)

A hollow fiber microfilter (BioOptimal (registered trademark) MF-SL, product of Asahi Kasei Medical) having a blocking pore size of 0.4 um, made of polysulfone, and having a gradient structure was prepared. A BioOptimal MF-SL minimodule was formed to have a membrane surface area of 3 cm$^2$. As in Patent Document 1 (Japanese Unexamined Patent Publication No. 2018-076291), the upstream side pore size, inner diameter, outer diameter, and thickness of the hollow fiber membrane of BioOptimal MF-SL were measured. As a result, the upstream side surface average pore size was 30 um or more and 80 $\mu$m or less, the inner diameter was 1.4 mm, the outer diameter was 2.3 mm, and the thickness was 0.45 mm.

As a cell culture solution for producing a raw material for a pharmaceutical material, a serum-free medium (IS CHO-CD medium, product of Irvine Scientific) on which monoclonal antibody-producing Chinese hamster ovary (CHO) cells had been cultured was prepared. The cell culture solution contained cells at a density of about $1.7 \times 10^6$ cells/mL and the viability of cells was about 83%.

To the cell culture solution, Triton X-100 (Merck) having a final concentration of 0.05%, Tween 20 (Molecular Biology Grade, Promega Corporation) having a final concentration of 0.2%, and CHPS (Fujifilm Wako Chemicals) having a final concentration of 0.5% were added, respectively, followed by incubation at 37°C for one hour to lyse the cells and cell culture solutions having reduced cell viability were obtained.

The BioOptimal MF-SL minimodule was fed with water by a peristaltic pump at a shear rate of 3000 /sec (48.5 mL/min) and a transmembrane pressure at that time was controlled to 20 kPa with a valve at the upstream side outlet of the hollow fiber membrane. While maintaining the transmembrane pressure by maintaining the state of the valve, water in the tube or module was removed. Then, the minimodule was fed with each of the cell culture solution and the cell culture solution having reduced cell viability by lysing cells and after a time lapse of 90 minutes or more, the permeability of an antibody was measured. The concentration of the antibody was measured with HPLC (Prominence, product of Shimadzu) by protein G column (PORPS G 20 $\mu$m, product of Thermo scientific). The results are shown in Table 2. The results show that in any case, the permeability of the antibody is high.

[Table 2]

| Viability before lysis | Surfactant | Viability after lysis | Membrane used | Permeability [%] of antibody |
| --- | --- | --- | --- | --- |
| 83% | - | - | MF-SL | 101 |

(continued)

| Viability before lysis | Surfactant | Viability after lysis | Membrane used | Permeability [%] of antibody |
|---|---|---|---|---|
| 83% | Triton 0.05% | 11% | MF-SL | 101 |
| 83% | Tween20 0.2% | 5% | MF-SL | 102 |
| 83% | CHAPS 0.5% | 14% | MF-SL | 98 |

(Referential Example 2)

A BioOptimal MF-SL minimodule similar to that of Referential Example 1 was created.

A serum-free medium (IS CHO=CD medium, product of Irvine Scientific) in which an antibody-producing Chinese hamster ovary (CHO) cells had been cultured was prepared as a cell culture solution for producing a raw material for a pharmaceutical material. The cell culture solution contained cells at a density of about $2.0 \times 10^6$ cells/mL and the viability of the cells was about 57%.

Triton X-100 (Merck) having a final concentration of 0.05% was added to the resulting cell culture solution, followed by incubation at 37°C for one hour to lyse the cells and thereby obtain a cell culture solution having reduced cell viability.

As in Referential Example 1, the minimodule was fed with the cell culture solution having cell viability reduced by lysing the cells. After a time lapse of 100 minutes or more, the permeability of the antibody was measured. The results are shown in Table 3. The results show that the permeability of the antibody is high.

[Table 3]

| Viability before lysis | Surfactant | Viability after lysis | Membrane used | Permeability [%] of antibody |
|---|---|---|---|---|
| 57% | Triton 0.05% | 10% | MF-SL | 99 |

(Referential Example 3)

A BioOptimal MF-SL minimodule similar to that of Referential Example 1 was created.

A serum-free medium (IS CHO-CD medium, product of Irvine Scientific) in which an antibody-producing Chinese hamster ovary (CHO) cells had been cultured was prepared as a cell culture solution for producing a raw material for a pharmaceutical material. The cell culture solution contained cells at a density of about $2.0 \times 10^6$ cells/mL and the viability of the cells was about 42%.

Triton X-100 (Merck) having a final concentration of 0.05% was added to the resulting cell culture solution, followed by incubation at 37°C for one hour to lyse the cells and thereby obtain a cell culture solution having reduced cell viability.

As in Referential Example 1, the minimodule was fed with the cell culture solution having cell viability reduced by lysing the cells. After a time lapse of 100 minutes or more, the permeability of the antibody was measured. The results are shown in Table 4. The results show that the permeability of the antibody is high.

[Table 4]

| Viability before lysis | Surfactant | Viability after lysis | Membrane used | Permeability [%] of antibody |
|---|---|---|---|---|
| 42% | Triton 0.05% | 7% | MF-SL | 94 |

(Referential Comparative Example 1)

A hollow fiber microfilter (MICROZA (registered trademark) UMP, product of Asahi Kasei) having a blocking pore size of 0.2 um, made of polyvinylidene fluoride (PVDF), and having a uniform structure was prepared. A MICROZA UMP minimodule was created to have a membrane surface area of 3 cm². The hollow fiber membrane having a uniform structure is substantially uniform throughout the hollow fiber membrane and is substantially uniform in the thickness direction of the hollow fiber membrane.

A hollow fiber microfilter (MICROZA (registered trademark) UJP) having a blocking pore size of 0.65 um, made of polyvinylidene fluoride (PVDF), and having a uniform structure was prepared. A MICROZA UJP minimodule was created to have a membrane surface area of 3 cm².

A cell culture solution and a cell culture solution having cell viability reduced by lysing the cells were prepared as in Referential Examples 2 and 3.

In a manner similar to that of Referential Examples 2 and 3 except for the use of the MICROZA UMP minimodule and the MICROZA UJP minimodule instead, the minimodules were each fed with the cell culture solution having cell viability reduced by lysing the cells and antibody permeability after a time lapse of 100 minutes or more was measured. The results are shown in Table 5.

[Table 5]

| Viability before lysis | Surfactant | Viability after lysis | Membrane used | Permeability [%] of antibody |
|---|---|---|---|---|
| 57% | Triton 0.05% | 10% | UMP | 86 |
| 57% | Triton 0.05% | 10% | UJP | 77 |
| 42% | Triton 0.05% | 7% | UMP | 61 |
| 42% | Triton 0.05% | 7% | UJP | 66 |

(Referential Example 4)

A BioOptimal MF-SL minimodule similar to that of Referential Example 1 was created.

A serum-free medium (IS CHO-CD medium, product of Irvine Scientific) in which antibody-producing Chinese hamster ovary (CHO) cells had been cultured was prepared as a cell culture solution for producing a raw material for a pharmaceutical material. The cell culture solution contained cells at a density of about $2.2 \times 10^6$ cells/mL and the viability of the cells was about 84%.

Triton X-100 (Merck) having a final concentration of 0.05% was added to the resulting cell culture solution, followed by incubation at 37°C for one hour to lyse the cells and thereby obtain a cell culture solution having reduced cell viability.

A transmembrane pressure was adjusted to 20 kPa with the valve of the upstream side outlet while feeding the module with 60 mL of the solution of the biopharmaceutical culture solution at a shear rate of 3000 /sec (48.5 mL/min). While keeping the transmembrane pressure, permeability of the antibody was measured after deadend filtration. The results are shown in Table 6.

(Referential Comparative Example 2)

MICROZA UMP and MICROZA UJP minimodules similar to those of Referential Comparative Example 1 were created.

As in Referential Example 4, a cell culture solution and a culture solution having cell viability reduced by lysing the cells were prepared.

In a manner similar to that of Referential Example 4 except for the use of the MICROZA UMP minimodule and the MICROZA UJP minimodule instead, the minimodules were each fed with the cell culture solution having cell viability reduced by lysing the cells and antibody permeability after a time lapse of 250 minutes or more was measured. The results are shown in Table 6.

[Table 6]

| Viability before lysis | Lysing method | Viability after lysis | Membrane used | Permeability [%] of antibody |
|---|---|---|---|---|
| 84% | Triton 0.05% | 4% | MF-SL | 100 |
| 84% | Triton 0.05% | 4% | UMP | 73 |
| 84% | Triton 0.05% | 4% | UJP | 74 |

(Referential Example 5)

A BioOptimal MF-SL minimodule similar to that of Referential Example 1 was created.

A serum-free medium (IS CHO-CD medium, product of Irvine Scientific) in which antibody-producing Chinese hamster ovary (CHO) cells had been cultured was prepared as a cell culture solution for producing a raw material for a pharmaceutical material. The cell culture solution contained cells at a density of about $2.2 \times 10^6$ cells/mL and the viability of the cells was about 2 6%. In addition, a similar culture solution containing cells at a density of about $2.0 \times 10^6$ cells/mL

and having viability of cells of about 13% was prepared.

Triton X-100 (Merck) having a final concentration of 0.05% was added to the respective cell culture solutions, followed by incubation at 37°C for one hour to lyse the cells and thereby obtain cell culture solutions having reduced cell viability.

A transmembrane pressure was adjusted to 20 kPa with the valve of the upstream side outlet while feeding the module with the solution of the biopharmaceutical culture solution at a shear rate of 3000 /sec (48.5 mL/min). While keeping the transmembrane pressure, the solution was filtered for 100 minutes or more and antibody permeability was then measured. The results are shown in Table 7.

(Referential Comparative Example 3)

MICROZA UMP and MICROZA UJP minimodules similar to those of Referential Comparative Example 1 were created.

As in Referential Example 5, a cell culture solution and a cell culture solution having cell viability reduced by lysing the cells were prepared.

In a manner similar to that of Referential Example 5 except for the use of the MICROZA UMP minimodule and the MICROZA UJP minimodule instead, the minimodules were each fed with the cell culture solution having cell viability reduced by lysing the cells and antibody permeability after a time lapse of 100 minutes or more was measured. The results are shown in Table 7.

[Table 7]

| Viability before lysis | Lysing method | Viability after lysis | Membrane used | Permeability [%] of antibody |
| --- | --- | --- | --- | --- |
| 26% | Triton 0.05% | 5% | MF-SL | 102 |
| 26% | Triton 0.05% | 5% | UMP | 65 |
| 26% | Triton 0.05% | 5% | UJP | 80 |
| 13% | Triton 0.05% | 5% | MF-SL | 102 |
| 13% | Triton 0.05% | 5% | UMP | 81 |
| 13% | Triton 0.05% | 5% | UJP | 81 |

(Referential Example 6)

A BioOptimal MF-SL minimodule similar to that of Referential Example 1 was created.

A serum-free medium (IS CHO-CD medium, product of Irvine Scientific) in which Chinese hamster ovary (CHO) cells had been cultured was prepared as a cell culture solution for producing a raw material for a pharmaceutical material. The cell culture solution contained cells at a density of about $2.3 \times 10^6$ cells/mL and the viability of the cells was about 69%.

Triton X-100 (Merck) having final concentrations of 0.05% and 0.1% and CHAPS (Fujifilm Wako Chemicals) having final concentrations of 0.5% and 1% were added to the resulting cell culture solution, respectively, followed by incubation at 37°C for one hour to lyse the cells and thereby obtain cell culture solutions having cell viability reduced to 15% or less.

A transmembrane pressure was adjusted to 20 kPa with the valve of the upstream side outlet while feeding the module with each of the solutions of the biopharmaceutical culture solution at a shear rate of 3000 /sec (48.5 mL/min) . While maintaining the transmembrane pressure, antibody permeability after a time lapse of 150 minutes or more was measured. The results are shown in Table 8.

[Table 8]

| Viability before lysis | Surfactant | Viability after lysis | Membrane used | Permeability [%] |
| --- | --- | --- | --- | --- |
| 69% | Triton 0.05% | 6% | MF-SL | 100 |
| 69% | Triton 0.1% | 8% | MF-SL | 99 |
| 69% | CHAPS 0.5% | 14% | MF-SL | 98 |
| 69% | CHAPS 1% | 3% | MF-SL | 100 |

(Experiment Example 1)

A BioOptimal MF-SL minimodule similar to that of Referential Example 1 and MICROZA UMP and MICROZA UJP minimodules similar to those of Referential Comparative Example 1 are created.

As a cell culture solution for producing a raw material for a pharmaceutical material, a medium having human embryonic kidney (HEK) 293 cells cultured therein is prepared.

After transfection of a plasmid DNA for producing AAV into cells and culturing the resulting cells for several days, TritonX-100 and CHAPS are added and the resulting mixtures are incubated at 37°C for one hour to lyse the cells and thereby obtain cell culture solutions having reduced cell viability.

A transmembrane pressure is adjusted to 20 kPa with the valve of the upstream side outlet while feeding, at a shear rate of 3000 /sec, the respective modules with the lysis solutions of the biopharmaceutical culture solution. While maintaining the transmembrane pressure, AAV permeability and a solution processing amount after deadend filtration or filtration for 100 minutes or more are measured.

(Experiment Example 2)

A BioOptimal MF-SL minimodule similar to that of Referential Example 1 and MICROZA UMP and MICROZA UJP minimodules similar to those of Referential Comparative Example 1 are created.

As a cell culture solution for producing a raw material for a pharmaceutical material, a medium having human embryonic kidney (HEK) 293 cells cultured therein is prepared.

A plasmid DNA for producing AAV is transfected into cells and the resulting cells are cultured for several days. Thus, a cell culture solution having reduced cell viability is prepared.

A transmembrane pressure is adjusted to 20 kPa with the valve of the upstream side outlet while feeding the respective modules with the lysis solution of the biopharmaceutical culture solution at a shear rate of 3000 /sec. While maintaining the transmembrane pressure, AAV permeability and a solution processing amount after deadend filtration or filtration for 100 minutes or more are measured.

(Experiment Example 3)

A BioOptimal MF-SL minimodule similar to that of Referential Example 1 and MICROZA UMP and MICROZA UJP minimodules similar to those of Referential Comparative Example 1 are created.

As a cell culture solution for producing a raw material for a pharmaceutical material, a medium having human embryonic kidney (HEK) 293 cells cultured therein is prepared.

After a plasmid DNA for producing AAV is transfected into cells and the resulting cells are cultured for several days, an acidic material is added and the resulting mixture is incubated at 37°C for one hour to lyse the cells. Thus, a cell culture solution having reduced cell viability is prepared.

A transmembrane pressure is adjusted to 20 kPa with the valve of the upstream side outlet while feeding, at a shear rate of 3000 /sec, the respective modules with the solution of the biopharmaceutical culture solution. While maintaining the transmembrane pressure, AAV permeability and a solution processing amount after deadend filtration or filtration for 100 minutes or more are measured.

(Example 1)

A BioOptimal MF-SL minimodule similar to that of Referential Example 1 and MICROZA UMP and MICROZA UJP minimodules similar to those of Referential Comparative Example 1 were created.

HEK293EB cells stably expressing the E1 gene region and Bcl-xL gene of an adenovirus were prepared. In a HYPERflask (Corning), the HEK293EB cells were cultured in a DMEM medium (Wako) mixed with 10% FBS and a 1% penicillin streptomycin solution. After culturing for 4 days, the cell culture solution was discarded and a plasmid DNA for producing AAV5 and a fluorescent protein ZsGreen was transfected into the HEK293EB cells. In the transfection, a transfection reagent was prepared by mixing 66.5 $\mu$g of a helper plasmid, 33.3 $\mu$g of a Rep/Cap plasmid, and 33.3 $\mu$g of GOI plasmid, which had been prepared in advance, with 399 $\mu$g of polyethyleneimine and allowing the resulting mixture to stand for 15 minutes. In the HYPERflask, the transfection reagent and a DMEM medium to which a 1% penicillin streptomycin solution, 1% GlutaMax, 0.1% NaHCO$_3$, and 0.1% D-Glucose had been added were poured and the plasmid DNA was transfected into the cells. After the transfection, the HEK293EB cells were cultured for 5 days. The viability of the HEK293EB cells in the culture solution decreased to 35%.

While the MF-SL modules were fed with 300 mL of a $4.8 \times 10^5$ cells/mL culture solution of the HEK293EB cells prepared in advance and the UMP and UJP modules were fed with 150 mL of the same solution prepared in advance, each fed at a shear rate of 3000 /sec, a transmembrane pressure was adjusted to 20 kPa with the valve of the upstream side outlet. While maintaining the transmembrane pressure, the culture solutions were filtered. The filtration processing amount of the culture solutions, the concentration ratio by filtration, and the permeability of AAV in the filtration were measured. The term "concentration ratio" as used herein means a ratio of a solution amount which has remained after filtration to a solution amount before filtration as shown in the following equation. As the concentration ratio is higher, the solution is concentrated.

Concentration ratio = (solution amount before filtration) / (solution amount which has remained after filtration.

As a result, as shown in Table 9, the processing amount of the culture solution when MF-SL was used is highest, the concentration ratio when MF-SL was used is highest, and AAV permeability when MF-SL was used is highest. In addition, under the conditions where the viability of the cells is as low as 35%, a solution having a cell density as high as $4.8 \times 10^5$ cells/mL can be filtered at a processing amount of 485 L/m$^2$ and AAV permeability of 96%, suggesting that even if the cell density is as high as about $1 \times 10^7$ cells/mL, filtration with high permeability can be performed.

[Table 9]

| Number of cells [cells/mL ] | Viabilit y | Membran e used | Shea r rate [s-1 ] | Processin g amount [L/m$^2$] | Concentratio n ratio | AAVpermeabilit y [%] |
|---|---|---|---|---|---|---|
| $4.8 \times 10^5$ | 35% | MF-SL | 3000 | 485 | 1.9 | 96 |
| $4.8 \times 10^5$ | 35% | UMP | 3000 | 92 | 1.4 | 4 |
| $4.8 \times 10^5$ | 35% | UJP | 3000 | 84 | 1.2 | 17 |

(Example 2)

A BioOptimal MF-SL minimodule similar to that of Referential Example 1 was created.
As in Example 1, HEK293EB cells were cultured and were transfected with a plasmid DNA for producing AAV1 and a fluorescent protein ZsGreen. After the transfection, the resulting HEK293EB cells were cultured for 5 days. The viability of the HEK293EB cells in the culture solution decreased to 10%.
To a portion of the HEK293EB cell culture solution were added a disodium salt of EDTA (2NA(EDTA·2Na), DO JINDO) having a final concentration of 0.1 M and Triton X-100 (SIGMA) having a final concentration of 0.05 (w/v) %. The cells were lysed for one hour. After the lysis, the viability of the HEK293EB cells in the medium decreased to 6%.
While the module was fed, at a shear rate of 3000 /sec and a flow rate of 48.5 mL/m, with an $8.4 \times 10^5$ cells/mL HEK293EB cell culture solution to which neither EDTA having a final concentration of 0.1 M nor Triton having a final concentration of 0.05 (w/v) % had been added, a transmembrane pressure was adjusted to 20 kPa with the valve of the upstream side outlet and the culture solution was filtered at the transmembrane pressure maintained thereat. The filtration processing amount of the culture solution, the concentration ratio by filtration, and the AAV permeability in filtration were measured.
While the module was fed, at a shear rate of 3000 /sec and a flow rate of 48.5 mL/m, with a $1.3 \times 10^6$ cells/mL HEK293EB cell culture solution to which EDTA having a final concentration of 0.1 M and Triton having a final concentration of 0.05 (w/v) % had been added, a transmembrane pressure was adjusted to 20 kPa with the valve of the primary side outlet and the culture solution was filtered at the transmembrane pressure maintained thereat. The filtration processing amount of the culture solution, the concentration ratio by filtration, and the AAV permeability in filtration were measured. It is to be noted that by the addition of EDTA, the cells which had attached to an incubator were suspended in the culture solution, leading to an increase in the number of the cells in the culture solution.
As a result, as shown in Table 10, the AAV permeability was higher when the cells were lysed with the surfactant before filtration. In addition, although the processing amount of the culture solution was lower when the surfactant was added, the AAV concentration in the culture solution was high due to the release of AAV from the cells when the surfactant was added. It is therefore presumed that the permeation amount of AAV by filtration is larger when the surfactant is added. Further, a solution having a cell density as high as $8.4 \times 10^5$ cells/mL was filtered at a processing amount of 374 L/m$^2$ and at 80% AAV permeability under the conditions where the viability of the cells was as low as 10%, suggesting that filtration at high permeability can be attained even if the cell density is as high as about $1 \times 10^7$ cells/mL. Still further, a solution having a cell density as high as $1.3 \times 10^6$ cells/mL was filtered at a processing amount of 280 L/m$^2$ and at 117% AAV permeability under the conditions where the viability of the cells was as low as 6%, suggesting that filtration at high permeability can be achieved even if the cell density is as high as about $1 \times 10^7$ cells/mL.

[Table 10]

| Number of cells [cells/mL] | Viability | Additive | Viability after lysis | Membrane used | Shear rate [s-1] | Processing amount [L/m²] | Concentration ratio | AAV permeability [%] |
|---|---|---|---|---|---|---|---|---|
| $8.4 \times 10^5$ | 10% | - | - | MF-SL | 300 0 | 374 | 4 | 80 |
| $1.3 \times 10^6$ | - | EDTA + 0.05% Triton | 6% | MF-SL | 300 0 | 280 | 2.1 | 117 |

(Example 3)

A BioOptimal MF-SL minimodule similar to that of Referential Example 1 was created.

As in Example 1, HEK293EB cells were cultured and were transfected with a plasmid for producing AAV1 and a fluorescent protein ZsGreen. After the transfection, the HEK293EB cells were cultured for 5 days. The viability of the HEK293EB cells in the culture solution decreased to 39%.

To a portion of the HEK293EB cell culture solution were added EDTA having a final concentration of 0.1 M and CHAPS having a final concentration of 0.5 (w/v) % and the cells were lysed for one hour. After the lysis using 0.5 (w/v) % CHAPS, the viability of the HEK293EB cells in the medium decreased to 13%.

To a portion of the HEK293EB cell culture solution were added EDTA having a final concentration of 0.1 M and Triton having a final concentration of 0.05 (w/v) % and the cells were lysed for one hour. After the lysis using 0.05 (w/v) % Triton, the viability of the HEK293EB cells in the medium decreased to 19%.

While the module was fed, at a shear rate of 3000 /sec and flow rate of 48.5 mL/m, with an $8.1 \times 10^5$ cells/mL HEK293EB cell culture solution to which neither EDTA nor CHAPS or Triton had been added, a transmembrane pressure was adjusted to 20 kPa with the valve of the upstream side outlet and the culture solution was filtered at the transmembrane pressure maintained thereat. The filtration processing amount of the culture solution, the concentration ratio by filtration, and the AAV permeability in filtration were measured.

While the module was fed, at a shear rate of 3000 /sec and flow rate of 48.5 mL/m, with a $1.3 \times 10^6$ cells/mL HEK293EB cell culture solution to which EDTA having a final concentration of 0.1 M and CHAPS having a final concentration of 0.5 (w/v) % had been added, a transmembrane pressure was adjusted to 20 kPa with the valve of the upstream side outlet and the culture solution was filtered at the transmembrane pressure maintained thereat. The filtration processing amount of the culture solution, the concentration ratio by filtration, and the AAV permeability in filtration were measured.

While the module was fed, at a shear rate of 3000 /sec and a flow rate of 48.5 mL/m, with a $1.4 \times 10^6$ cells/mL HEK293EB cell culture solution to which EDTA and 0.05 (w/v) % Triton had been added, a transmembrane pressure was adjusted to 20 kPa with the valve of the upstream side outlet and the culture solution was filtered at the transmembrane pressure maintained thereat. The filtration processing amount of the culture solution, the concentration ratio by filtration, and the AAV permeability in filtration were measured.

The results are shown in Table 11. Since AAV was released more from the cells when the surfactant was added and the AAV concentration in the medium was higher when the surfactant was added, it is presumed that the amount of AAV permeated by filtration is larger when the surfactant is added. In addition, a solution having a cell density as high as $1.4 \times 10^6$ cells/mL was filtered at a processing amount of 217 L/m² and at 90% AAV permeability under the conditions where the viability of the cells was as low as 19%, suggesting that filtration at high permeability can be achieved even if the cell density is as high as about $1 \times 10^7$ cells/mL.

[Table 11]

| Number of cells [cells/mL] | Viability | Additive | Viability after lysis | Membrane used | Shear rate [s-1] | Processing amount [L/m²] | Concentration ratio | AAV permeability [%] |
|---|---|---|---|---|---|---|---|---|
| $8.1 \times 10^5$ | 39% | - | - | MF-SL | 300 0 | 393 | 4.7 | 89 |
| $1.3 \times 10^6$ | - | EDTA + 0.5% CHAPS | 13% | MF-SL | 300 0 | 203 | 1.5 | 83 |

(continued)

| Number of cells [cells/ mL ] | Viabilit y | Additiv e | Viability after lysis | Membran e used | Shea r rate [s-1 ] | Processin g amount [L/m²] | Concentrati on ratio | AAV permeabili ty [%] |
|---|---|---|---|---|---|---|---|---|
| $1.4 \times 10^6$ | | EDTA + 0.05% Trito n | 19% | MF-SL | 300 0 | 217 | 1.6 | 90 |

(Example 4)

A BioOptimal MF-SL minimodule similar to that of Referential Example 1 and MICROZA UMP and MICROZA UJP minimodules similar to those of Referential Comparative Example 1 were created.

As in Example 1, HEK293EB cells were cultured and transfected with a plasmid DNA for producing AAV2 and a fluorescent protein ZsGreen. After the transfection, the resulting HEK293EB cells were cultured for 5 days. To the HEK293EB cell culture solution was added 0.05 (w/v) % Triton and the cells were lysed for one hour.

While the aforesaid modules were each fed with the HEK293EB cell culture solution at a shear rate of 5000 /sec, the transmembrane pressure was adjusted to 20 kPa with the valve of the upstream side outlet and the culture solution was filtered at the transmembrane pressure maintained thereat. The filtration processing amount of the culture solution, the concentration ratio by filtration, and the AAV permeability in filtration were measured.

As a result, as shown in Table 12, the culture solution processing amount when MF-SL was used was the highest, the concentration ratio when MF-SL was used was the highest, and the AAV permeability when MF-SL was used was the highest.

[Table 12]

| Surfactan t | Membrane used | Shear rate [s-1] | Processing amount [L/m²] | Concentrati on ratio | AAV permeability [%] |
|---|---|---|---|---|---|
| 0.05% Triton | MF-SL | 5000 | 195 | 2.4 | 64 |
| 0.05% Triton | UMP | 5000 | 64 | 1.2 | 3 |
| 0.05% Triton | UJP | 5000 | 64 | 1.2 | 6 |

(Example 5)

A BioOptimal MF-SL minimodule similar to that of Referential Example 1 was created.

A first depth filter and a second depth filter were prepared. The first depth filter has a pore size of 2 um to 20 $\mu$m, is made of cellulose, dematiaceous earth, or the like, and has a membrane surface area of 0.002 m². The second depth filter has a pore size of 0.4 um to 1.0 $\mu$m, is made of cellulose, and has a membrane surface area of 0.002 m².

HEK293EB cells stably expressing the E1 gene region and Bcl-xL gene of an adenovirus were prepared. HEK293EB cells were cultured as in Example 1 and were transfected with a plasmid DNA for producing AAV1 and a fluorescent protein ZsGreen. After the transfection, the resulting HEK293EB cells were cultured for 5 days.

While the MS-FL minimodule was fed with the HEK293EB cell culture solution at a shear rate of 3000 /sec and a flow rate of 48.5 mL/min, a transmembrane pressure was adjusted to 20 kPa with the valve of the upstream side outlet and the culture solution was filtered at the transmembrane pressure maintained thereat.

The HEK293EB cell culture solution was poured at 3.7 mL/min to the first and second depth filters used in combination and the culture solution was subjected to batch filtration.

The results are shown in Table 13. The initial amount is an amount of the culture solution before filtration. The processing time is a filtration time. The filtration amount is an amount of the culture solution filtered. It is to be noted that the turbidity of each of the filtered solutions was 1 NTU or less.

[Table 13]

| | Target to be filtered | Initial amount | Processing time | Filtration amount | Processing amount |
|---|---|---|---|---|---|
| MF-SL | Culture solution | 150 g | 120 min | 130 g | 430 L/m² |
| Depth membrane | Culture solution | 440 g | 130 min | 440 g | 200 L/m² |

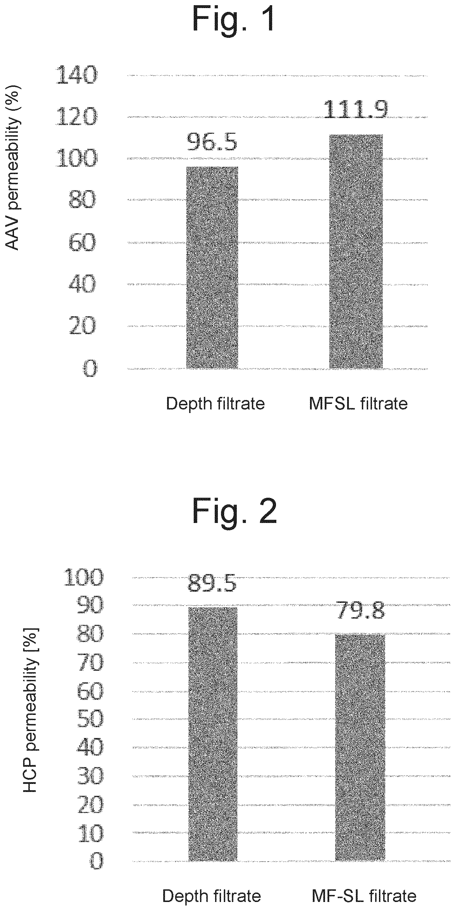

As shown in Fig. 1, the AAV permeability was higher in the filtration through the MF-SL minimodule than that through the depth membranes. As shown in Fig. 2, the permeability of a host-cell-derived protein (HCP) which was an impurity was lower in the filtration through the MF-SL minimodule than in the filtration through the depth membranes. This suggests that compared with the depth membranes, the hollow fiber membrane has a possibility of removing an impurity and permeating only an intended product.

(Example 6)

As in Example 1, HEK293EB cells were cultured and transfected with a plasmid DNA for producing AAV1 and a fluorescent protein ZsGeen. After the transfection, the resulting HEK293EB cells were cultured for 5 days. After culturing, the viability of the HEK293EB cells in the culture solution decreased to 35%.

While a hollow fiber microfilter (BioOptimal (registered trademark) MF-SL0005 (50 cm$^2$), product of Asahi Kasei Medical) was fed with 2 L of a $7.4 \times 10^5$ cells/mL HEK293EB cell culture solution at a flow rate of 100 mL/min, a transmembrane pressure was adjusted to 10 to 50 kPa with the valve of the upstream side outlet and the culture solution was filtered. After deadend filtration, extrusion washing was performed with 200 mL of a buffer (50 mM HEPES, 150 mM NaCl, and 1 mM MgCl$_2$) to recover the AAV which had remained in the hollow fiber or tube. The filtration processing amount of the culture solution, the concentration ratio by filtration, the AAV permeability in filtration, and the recovery percent of AAV including extrusion washing were measured. The recovery percent is represented by the following equation:

Recovery percent X= { (virus concentration in permeate) $\times$ amount of permeate} / { (virus concentration in cell culture solution before filtration) $\times$ amount of culture solution} $\times$ 100

As a result, the recovery percent increased to 100% as shown in Table 14. The AAV which has remained in the tube or membrane is recovered by extrusion washing. This means that AAV does not adsorb to the MF-SL and shows a good recovery percent. This suggests that it is worth using industrially.

[Table 14]

| Membrane used | Processing amount [L/m$^2$] | Concentration ratio | AAV permeability [%] | AAV recovery percent [%] |
|---|---|---|---|---|
| MF-SL | 400 | 40 | 95 | 100 |

(Experiment Example 4)

A BioOptimal MF-SL minimodule similar to that of Referential Example 1 is created.

As in Example 1, HEK293EB cells are cultured and are transfected with a plasmid DNA for producing AAV2 and a fluorescent protein ZsGreen. After the transfection, the resulting HEK293EB cells are cultured for 5 days.

An EDTA disodium salt (2NA(EDTA.2Na), DOJINDO) having a final concentration of 0.1 M is added to an incubator to peel off the HEK293EB cells. The cell solution thus obtained is centrifuged and pelletized. Triton X-100 (SIGMA) having a final concentration of 0.05 (w/v) % is added to the resulting cells to lyse the cells for one hour. After the lysis, the viability of the HEK293EB cells in the medium decreases to 10% or less.

While the module is fed, at a shear rate of 3000 /sec and a flow rate of 48.5 mL/m, with a $2.0 \times 10^7$ cells/mL HEK293EB cell culture solution to which EDTA having a final concentration of 0.1 M and Triton having a final concentration of 0.05 (w/v) % have been added, a transmembrane pressure is adjusted to 20 kPa with the valve of the upstream side outlet and the culture solution is filtered at the transmembrane pressure maintained thereat. The filtration processing amount of the culture solution, the concentration ratio by filtration, and the AAV permeability in filtration are measured.

(Referential Experiment Example 1)

A hollow fiber microfilter (BioOptimal (registered trademark) MF-SL, product of Asahi Kasei Medical) having a blocking pore size of 0.4 um, made of polysulfone, and having a gradient structure was prepared. A BioOptimal MF-SL minimodule was created to have a membrane surface area of 3 cm$^2$.

As a cell culture solution for producing a raw material for a pharmaceutical material, a serum-free medium in which Chinese hamster ovary (CHO) cells had been cultured was prepared. The cell culture solution contained the cells at a density of $1.7 \times 10^6$ cells/mL and the viability of the cells was about 83%.

To the cell culture solution were added Triton X-100 (Merck) having a final concentration of 0.05%, Tween 20 (Molecular Biology Grade, product of Promega) having a final concentration of 0.2%, and CHAPS (Fujifilm Wako Chemicals) having a final concentration of 0.5%, respectively, and the resulting mixtures were incubated at 37°C for one hour to lyse the cells. Thus, cell culture solutions having reduced cell viability were prepared.

The BioOptimal MF-SL minimodule was fed with water by a peristaltic pump at a shear rate of 3000 /sec (48.5 mL/min) and a transmembrane pressure at that time was adjusted to 20 kPa with a valve at the upstream side outlet of the hollow fiber membrane. While maintaining the state of the valve and maintaining the transmembrane pressure, water in the tube or module was removed. Then, the minimodule was fed with each of the cell culture solution and the cell culture solution having reduced cell viability and a solution processing amount per membrane surface area was calculated based on a permeation amount at the time of 100-min filtration. The permeate amount was measured with a gravimeter as needed. The turbidity of the solution was measured with a portable turbidimeter (Portable turbidimeter 210Q, product of Fuji Tecom) . The results are shown in Table 15. Even a reduction in the viability of cells did not cause a marked reduction in the processing amount.

[Table 15]

| Viability before lysis | Turbidity [NTU] before lysis | Surfactant | Viability after lysis | Turbidity [NTU] after lysis | Membrane used | Processing amount [L/m$^2$] | Turbidity [NTU] after filtration |
|---|---|---|---|---|---|---|---|
| 83% | 116 | - | - | - | MF-SL | 476 | 3.71 |
| 83% | 116 | Triton 0.05% | 11% | 18.7 | MF-SL | 521 | 1.93 |
| 83% | 116 | Tween20 0.2% | 5% | 42.7 | MF-SL | 351 | 12.3 |
| 83% | 116 | CHAPS 0.5% | 14% | 20.5 | MF-SL | 548 | 3.15 |

(Referential Example 1)

Batch filtration of a cell culture solution containing cells at a density of from about $11\times10^6$ cells/mL to $18.5\times10^6$ cells/mL was performed using a 0.005m$^2$ BioOptimal MF-SL at a shear rate of 3000 /sec without controlling a transmembrane pressure with a valve or the like. Then, the processing amount of the cell culture solution having the viability of cells of 99.5% was about 300 L/m$^2$, while the processing amount of the cell culture solution having the viability of cells reduced even to 26% by prolonging a culture time markedly decreased to 60 L/m$^2$, about one-sixth of that of the cell culture solution described above. Thus, in Referential Example 1, filterability markedly decreased when the viability of the cell culture solution was low as a result of culturing for long hours. The results are shown in Table 16. Permeation of cells having a density of from about $1\times10^7$ cells/mL to about $2\times10^7$ cells/mL was however allowed. The cell culture solution having a density as high as about $1\times10^7$ cells/mL to about $2\times10^7$ cells/mL was filtered at a processing amount of 60 L/m$^2$ under the conditions where the viability of cells was as low as 26%, suggesting that even if the cell density is as high as about $1\times10^7$ cells/mL, the MF-SL can filter such a solution.

[Table 16]

| Viability before lysis | Membrane used | Processing amount |
|---|---|---|
| 99.5% | MF-SL | 300 |
| 87.2% | MF-SL | 120 |
| 26% | MF-SL | 60 |

(Referential Experiment Example 2)

A BioOptimal MF-SL minimodule similar to that of Referential Experiment Example 1 was created.

A hollow fiber microfilter (MICROZA (registered trademark) UJP, product of Asahi Kasei) having a blocking pore size of 0.65 um, made of polyvinylidene fluoride (PVDF), and having a uniform structure was prepared. The MICROZA UJP minimodule was created to have a membrane surface area of 3 cm$^2$.

As a cell culture solution for producing a raw material for a pharmaceutical material, a serum-free medium (IS CHO-CD medium, product of Irvine Scientific) in which Chinese hamster ovary (CHO) cells had been cultured was prepared. The cell culture solution contained cells at a density of about $2.0\times10^6$ cells/mL and the viability of cells was about 57%.

Triton X-100 (Merck) having a final concentration of 0.05% was added to the cell culture solution and the resulting mixture was incubated at 37°C for one hour to lyse the cells and thereby obtain a cell culture solution having cell viability reduced to 10%.

The modules thus created were fed with water at a shear rate of 3000 /sec (MF-SL: 48.5 mL/min. UJP: 23.5 mL/min) and a transmembrane pressure at that time was adjusted to 20 kPa with the valve of the upstream side outlet of the hollow fiber membrane. After water in the tube and the module was removed while maintaining the state of the valve and maintaining the transmembrane pressure, the minimodules were each fed with the cell culture solution having reduced cell viability and from the permeate amount at the time of 100-min filtration, a solution processing amount per membrane surface area was measured. The results are shown in Table 17.

[Table 17]

| Viabilit y before lysis | Surfactan t | Viabilit y after lysis | Turbidit y [NTU] after lysis | Membran e used | Processin g amount [L/m$^2$] | Turbidity [NTU] after filtratio n |
|---|---|---|---|---|---|---|
| 57% | Triton 0.05% | 10% | 29.5 | MF-SL | 540 | 2.51 |
| 57% | Triton 0.05% | 10% | 29.5 | UJP | 120 | 1.94 |

(Referential Experiment Example 3)

As a cell culture solution for producing a raw material for a pharmaceutical material, a serum-free medium (IS CHO-CD medium, product of Irvine Scientific) in which Chinese hamster ovary (CHO) cells had been cultured was prepared. The cell culture solution contained cells at a density of about $2.0 \times 10^6$ cells/mL and the viability of the cells was about 42%.

Triton X-100 (Merck) having a final concentration of 0.05% was added to the cell culture solution and the resulting mixture was incubated at 37°C for one hour to lyse cells and thereby obtain a cell culture solution having cell viability reduced to 7%.

In a manner similar to that of Referential Experiment Example 2, a solution processing amount per membrane surface area was measured. The results are shown in Table 18.

[Table 18]

| Viability before lysis | Surfactant | Viability after lysis | Membrane used | Processing amount [L/m$^2$] |
|---|---|---|---|---|
| 42% | Triton 0.05% | 7% | MF-SL | 346 |
| 42% | Triton 0.05% | 7% | UJP | 54 |

(Referential Experiment Example 4)

A BioOptimal MF-SL minimodule similar to that of Referential Experiment Example 1 was created.

As a cell culture solution for producing a raw material for a pharmaceutical material, a serum-free medium (IS CHO-CD medium, product of Irvine Scientific) in which Chinese hamster ovary (CHO) cells had been cultured was prepared. The cell culture solution contained cells at a density of about $1.3 \times 10^6$ cells/mL and the viability of the cells was about 26%.

Triton X-100 (Merck) having a final concentration of 0.05% was added to the cell culture solution and the resulting mixture was incubated at 37°C for one hour to lyse cells and thereby obtain a cell culture solution having cell viability reduced to 5%.

While the module was fed with the solution of the biopharmaceutical culture solution at a shear rate of 3000 /sec (48.5 mL/min), a transmembrane pressure was adjusted to 20 kPa with the valve of the upstream side outlet and the solution processing amount per membrane surface area was calculated from the permeate amount at the time of 100-min filtration. The results are shown in Table 19.

(Referential Experiment Example 5)

As a cell culture solution for producing a raw material for a pharmaceutical material, a serum-free medium (IS CHO-CD medium, product of Irvine Scientific) in which Chinese hamster ovary (CHO) cells had been cultured was prepared. The cell culture solution contained cells at a density of about $1.3 \times 10^6$ cells/mL and the viability of the cells was about 13%. As in Referential Experiment Example 4, a solution processing amount per membrane surface area was measured. The results are shown in Table 19.

[Table 19]

| Viabili ty before lysis | Turbidi ty [NTU] before lysis | Surfacta nt | Viabili ty after lysis | Turbidi ty [NTU] after lysis | Membra ne used | Process! ng amount [L/m$^2$] | Turbidit y [NTU] after filtrati on |
|---|---|---|---|---|---|---|---|
| 26% | 99 | Triton 0.05% | 5% | 45.2 | MF-SL | 425 | 4.66 |
| 13% | 122 | Triton 0.05% | 5% | 89.1 | MF-SL | 389 | 4.21 |

(Referential Experiment Example 6)

A BioOptimal MF-SL minimodule similar to that of Referential Experiment Example 1 was created.

As a cell culture solution for producing a raw material for a pharmaceutical material, a serum-free medium (IS CHO-CD medium, product of Irvine Scientific) in which Chinese hamster ovary (CHO) cells had been cultured was prepared. The cell culture solution contained cells at a density of about $2.3 \times 10^6$ cells/mL and the viability of the cells was about 69%.

Triton X-100 (Merck) having final concentrations of 0.05% and 0.1% and CHPS (Fujifilm Wako Chemicals) having final concentrations of 0.5% and 1% were added to the cell culture solution and the resulting mixtures were incubated at 37°C for one hour to lyse cells and thereby obtain cell culture solutions having cell viability reduced to 15% or less, respectively.

While the module was fed with each of the solutions of the biopharmaceutical culture solution at a shear rate of 3000 /sec (48.5 mL/min), a transmembrane pressure was adjusted to 20 kPa with the valve of the upstream side outlet. At the transmembrane pressure maintained thereat, the solution processing amount per membrane surface area was calculated from the permeate amount at the time of 150-min filtration. The results are shown in Table 20.

[Table 20]

| Viabili ty before lysis | Turbidi ty [NTU] before lysis | Surfacta nt | Viabili ty after lysis | Turbidi ty [NTU] after lysis | Membra ne used | Process! ng amount [L/m$^2$] | Turbidit y [NTU] after filtrati on |
|---|---|---|---|---|---|---|---|
| 69% | 102 | Triton 0.05% | 6% | 38.1 | MF-SL | 808 | 3.62 |
| 69% | 102 | Triton 0.1% | 8% | 35.9 | MF-SL | 854 | 3.35 |
| 69% | 102 | CHAPS 0.5% | 14% | 34.9 | MF-SL | 576 | 6.23 |
| 69% | 102 | CHAPS 1% | 3% | 32 | MF-SL | 549 | 4.11 |

(Referential Experiment Example 7)

A BioOptimal MF-SL minimodule similar to that of Referential Experiment Example 1 was created. In addition, a MICROZA UJP minimodule similar to that of Referential Experiment Example 2 was created.

As a cell culture solution for producing a raw material for a pharmaceutical material, a serum-free medium (CHO-CD medium, product of Irvine Scientific) in which Chinese hamster ovary (CHO) cells had been cultured was prepared. The cell culture solution contained cells at a density of about $1.3 \times 10^6$ cells/mL and the viability of the cells was about 13%.

Triton X-100 (Merck) having a final concentration of 0.05% was added to the cell culture solution was added and the resulting mixture was incubated at 37°C for one hour to lyse the cells and thereby obtain a cell culture solution having cell viability reduced to 10%.

The modules thus created were each fed with 60 ml of the cell culture solution having reduced cell viability at a shear rate of 3000 /sec (MF-SL: 48.5 mL/min. UJP: 23.5 mL/min) and a transmembrane pressure at that time was adjusted to 20 kPa with the valve of the upstream side outlet of the hollow fiber membrane. While maintaining the state of the valve and maintaining the transmembrane pressure, the minimodules were each fed with the cell culture solution having reduced cell viability and from the permeate amount at the time of deadend filtration or at the time of 100-min filtration, a solution processing amount per membrane surface area was measured. The results are shown in Table 21.

[Table 21]

| Viability before lysis | Surfactant | Viability after lysis | Membrane used | Processing amount [L/m$^2$] |
|---|---|---|---|---|
| 84% | Triton 0.05% | 4% | MF-SL | 172 |
| 84% | Triton 0.05% | 4% | UJP | 139 |

(Referential Experiment Example 8)

A BioOptimal MF-SL minimodule similar to that of Referential Experiment Example 1 was created. In addition, a MICROZA UJP minimodule similar to that of Referential Experiment Example 2 was created.

As a cell culture solution for producing a raw material for a pharmaceutical material, a medium in which human embryonic kidney (HEK) 293 cells (293AAV, Cell Biolabs) had been cultured was prepared. The cell culture solution contained cells at a density of about $2.0 \times 10^6$ cells/mL.

Triton X-100 (Sigma-Aldrich) having a final concentration of 0.05% was added to the cell culture solution and the resulting mixture was incubated at 37°C for one hour to lyse the cells and thereby obtain a cell culture solution having reduced cell viability.

KrosFlo KR2i TFF System (Repligen) was used for the modules and the shear rate was adjusted to 3000 /sec (MF-SL: 48.5 mL/min. UJP: 23.5 mL/min) and the transmembrane pressure was adjusted to 20 kPa with the valve of the upstream side outlet of the hollow fiber membrane. The opening or closing condition of the valve is automatically controlled so as to keep the transmembrane pressure at 20 kPa. The minimodules were each fed with about 100 mL of the cell culture solutions having reduced cell viability and from the permeate amount at the time of deadend filtration or at the time of 120-min filtration, a solution processing amount per membrane surface area was measured. The processing amount was 340 L/m$^2$ for MF-SL and 140 L/m$^2$ for UJP.

**Claims**

1. A virus recovery method, comprising:

   reducing viability of cells in a culture solution, and
   filtering, through a hollow fiber membrane, the culture solution containing a virus produced by the cells to recover the virus, wherein:
   the hollow fiber membrane has a gradient structure in which an average pore size becomes smaller from an upstream side to a downstream side in a membrane thickness direction.

2. The virus recover method according to claim 1, wherein the hollow fiber membrane has a pore size of 20 pm or more and 100 um or less on the upstream side.

3. The virus recovery method according to claim 1, wherein a pore of the hollow fiber membrane has a blocking pore size of 0.05 um or more and 20 $\mu$m or less.

4. The virus recovery method according to claim 1, wherein the hollow fiber membrane has a synthetic polymer.

5. The virus recovery method according to claim 4, wherein the synthetic polymer is polysulfone.

6. The virus recovery method according to claim 1, wherein the hollow fiber membrane has a coarse layer and a dense layer.

7. The virus recovery method according to claim 1, wherein in reducing viability of the cells, the viability of the cells is reduced by chemical processing or physical processing.

8. The virus recovery method according to claim 1, wherein in reducing viability of the cells, a chemical material is brought into contact with the cells.

9. The virus recovery method according to claim 8, wherein the chemical material is a surfactant, an acidic material, or a basic material.

**10.** The virus recovery method according to claim 9, wherein the cells are lysed by the surfactant.

**11.** The virus recovery method according to claim 9, wherein the surfactant is a nonionic surfactant or an amphoteric surfactant.

**12.** The virus recovery method according to claim 1, wherein in reducing viability of the cells, transfection into the cells is performed.

**13.** The virus recovery method according to claim 1, wherein after reduction of the viability of the cells, the viability of the cells is 60% or less.

**14.** The virus recovery method according to claim 1, wherein after reduction of the viability of the cells, the viability of the cells is 30% or less.

**15.** The virus recovery method according to claim 1, not substantially comprising, before the filtering, removing the cells and/or debris of the cells with a precipitating medium.

**16.** The virus recovery method according to claim 1, wherein the virus is an adeno-associated virus.

# Fig. 1

# Fig. 2

## INTERNATIONAL SEARCH REPORT

| International application No. |
| --- |
| **PCT/JP2022/026434** |

**A. CLASSIFICATION OF SUBJECT MATTER**

*C12N 7/02*(2006.01)i; *B01D 61/00*(2006.01)i; *B01D 69/08*(2006.01)i; *C12N 1/00*(2006.01)i; *C12N 1/02*(2006.01)i
FI:  C12N7/02; B01D61/00; B01D69/08; C12N1/00 K; C12N1/02

According to International Patent Classification (IPC) or to both national classification and IPC

**B. FIELDS SEARCHED**

Minimum documentation searched (classification system followed by classification symbols)

C12N7/02; B01D61/00; B01D69/08; C12N1/00; C12N1/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2007-295937 A (ONCOLYTICS BIOTECH INC) 15 November 2007 (2007-11-15) paragraphs [0010]-[0012], [0017]-[0018], [0038], [0046], [0049] | 1-16 |
| Y | JP 2018-76291 A (ASAHI KASEI MEDICAL CO LTD) 17 May 2018 (2018-05-17) claims 1, 8, 10-13, 16, paragraph [0010] | 1-16 |
| Y | JP 2004-525755 A (AMERSHAM BIOSCIENCES PATENT DEPARTMENT 800 CENTENNIAL AVENUE PISCATAWAY) 26 August 2004 (2004-08-26) claims 1, 4, paragraphs [0046], [0073], [0074], fig. 1 | 1-16 |
| Y | WO 2010/035793 A1 (ASAHI KASEI CHEMICALS CORPORATION) 01 April 2010 (2010-04-01) paragraphs [0016], [0023], [0025], [0042] | 1-16 |
| Y | JP 2020-121299 A (ASAHI KASEI MEDICAL CO LTD) 13 August 2020 (2020-08-13) claims 1, 7, 10-12, paragraphs [0044], [0056] | 1-16 |
| Y | WO 2020/023612 A1 (VOYAGER THERAPEUTICS, INC.) 30 January 2020 (2020-01-30) paragraph [0171] | 12 |

☐ Further documents are listed in the continuation of Box C.　　☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **01 August 2022** | **16 August 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2022/026434**

| Patent document cited in search report | | | Publication date (day/month/year) | Patent family member(s) | | | Publication date (day/month/year) |
|---|---|---|---|---|---|---|---|
| JP | 2007-295937 | A | 15 November 2007 | US 2005/0095692 A1 paragraphs [0027]-[0032], [0037]-[0038], [0058], [0067], [0070] EP 2253701 A1 | | | |
| JP | 2018-76291 | A | 17 May 2018 | (Family: none) | | | |
| JP | 2004-525755 | A | 26 August 2004 | US 2004/0050791 A1 claims 1, 4, paragraphs [0050], [0077]-[0078], fig. 1 | | | |
| WO | 2010/035793 | A1 | 01 April 2010 | US 2011/0210067 A1 paragraphs [0019]-[0021], [0024], [0036], [0038], [0055] EP 2335814 A1 CN 102164657 A | | | |
| JP | 2020-121299 | A | 13 August 2020 | (Family: none) | | | |
| WO | 2020/023612 | A1 | 30 January 2020 | JP 2021-530548 A US 2021/0355454 A1 CN 112770812 A | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018076291 A
- JP 6530171 B
- JP 2010035793 A
- JP 2020023612 A